(19) Europäisches Patentamt
European Patent Office
Office européen des brevets

(11) Publication number: **0 061 264**
**B1**

(12) # EUROPEAN PATENT SPECIFICATION

(45) Date of publication of patent specification: **24.07.85**

(21) Application number: **82301256.2**

(22) Date of filing: **11.03.82**

(51) Int. Cl.$^4$: **C 07 C 121/64,** C 09 B 1/00, H 01 B 1/12, H 01 L 29/28, C 08 G 61/10, C 08 G 65/34, C 08 K 5/29

(54) **Tetracyanoanthraquinodimethane compounds and processes for the production thereof, polymers and charge-transfer complexes derived therefrom.**

(30) Priority: **13.03.81 JP 36728/81**
**10.07.81 JP 108593/81**

(43) Date of publication of application:
**29.09.82 Bulletin 82/39**

(45) Publication of the grant of the patent:
**24.07.85 Bulletin 85/30**

(84) Designated Contracting States:
**DE FR GB**

(56) References cited:
**BE-A- 668 658**
**FR-A-2 211 464**
**US-A-3 097 227**
**US-A-3 687 987**

**CHEMICAL ABSTRACTS, vol. 91, no. 1, 2nd July 1979, page 456, no. 4846g, Columbus Ohio (USA); E.F. McINTYRE et al.: "Calculation of nitrogen-containing heterocycles and of nitriles"**

(73) Proprietor: **Matsushita Electric Industrial Co., Ltd.**
**1006, Oaza Kadoma**
**Kadoma-shi Osaka-fu, 571 (JP)**

(72) Inventor: **Hhota, Shu**
**8-46, Kagucho 1-chome Nagao**
**Hirakata-shi Osaka-fu (JP)**
Inventor: **Tosaka, Tomiharu**
**34-7, Yawatakonishi**
**Yawata-shi Kyoto-fu (JP)**
Inventor: **Sonoda, Nobuo**
**16-5, Torikainaka 1-chome**
**Settsu-shi Osaka-fu (JP)**
Inventor: **Shimotsuma, Wataru**
**12-8, Yamatedai 5-chome**
**Ibaragi-shi Osaka-fu (JP)**

(74) Representative: **Spencer, Graham Easdale et al**
**A.A. Thornton & Co Northumberland House**
**303-306, High Holborn**
**London WC1V 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 061 264 B1

**Description**

This invention relates to novel compounds, and more particularly to 11,11,12,12-tetracyano-9,10-anthraquinodimethane and its derivatives.

Heretofore, 7,7,8,8-tetracyanoquinodimethane (hereinafter referred to briefly as TCNQ) and 9,9,10,10-tetracyano-1,4-naphthaquinodimethane (briefly, TCNNQ) and various derivatives thereof (All of these compounds are collectively referred to as TCNQ and TCNNQ compounds) are known. These organic compounds have excellent semi-conductor characteristics and, as is well known, have great industrial values.

The molecular formulas of TCNQ and TCNNQ are presented hereinafter [(1) and (2), respectively]. TCNQ and TCNNQ, and various derivatives thereof have the tetracyanoquinodimethane skeleton represented by formula (3), and this skeletal structure is determinant of the characteristics of these compounds.

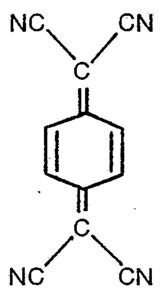

(1)

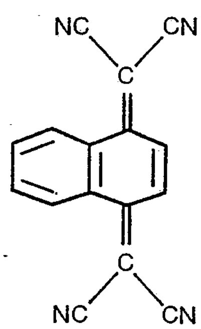

(2)

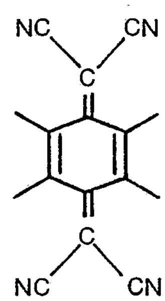

(3)

In this specification and the claims appended thereto, the term 'tetracyanoquinodimethane skeleton' means the skeletal structure consisting of those carbon and nitrogen atoms which constitute the molecule of TCNQ.

TCNQ and its derivatives have been described in the literature including D. S. Acker, et al., J. Am. Chem. Soc., *84*, 3370 (1962) and R. C. Wheland, et al., J. Org. Chem., *40* (21), 3101 (1975).

The structural analysis of TCNNQ has been attempted and reported in F. Iwasaki, Acta Cryst. B*27*, 1360 (1971).

Summary of the Invention

This invention provides novel compounds which, based on the tetracyanoquinodimethane skeleton possessed in common, display certain characteristics which are not seen in TCNQ or TCNNQ or in any known derivative thereof.

The novel organic compounds according to this invention are 11,11,12,12-tetracyano-9,10-anthraquinodimethane and its derivatives (hereinafter referred to as TCNAQ compounds), and may be represented by the following general formula (A).

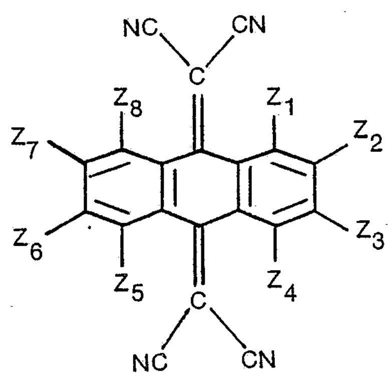

(A)

wherein each of $Z_1$, $Z_4$, $Z_5$ and $Z_8$ is H, Cl, hydroxy or amino and each of $Z_2$, $Z_3$, $Z_6$ and $Z_7$ is H, halogen, alkyl, phenyl, alkylphenyl, hydroxyalkyl, carboxyalkyl, hydroxy, amino or carboxy, said alkyl, alkylphenyl, hydroxyalkyl and carboxyalkyl groups each containing up to 8 carbon atoms.

The halogen referred to above may be fluorine, chlorine, bromine or iodine.

A variety of TCNAQ compounds having useful characteristics can be obtained by selecting suitable

species for substituents $Z_1$ to $Z_8$. The compounds can be easily produced from benzoquinone and butadiene derivatives, as will be described hereinafter.

The TCNAQ compounds according to the invention have a tetracyanoanthraquinodimethane skeleton; this skeleton has the following features.

(a) The skeleton has a tetracyanoquinodimethane nucleus;

(b) Substituents can be present at any of positions 1 to 8.

(c) The skeleton has an aromatic benzene ring at each side of the nucleus.

The semiconductor characteristics of TCNAQ compounds result from its tetracyanoquinodimethane structure (a). The other features (b) and (c) each provides basis for the versatility and functionality of the derivatives as organic compounds, and are the features which differentiate TCNAQ compounds from TCNQ and TCNNQ compounds.

Particularly, the tetracyanoanthraquinodimethane skeleton has a large expanse of electron cloud and due to the synergistic action of the tetracyanoquinodimethane nucleus and the benzene rings fused thereto, the energy breadth between the ground and excited states of electrons is so small that some useful results such as increased electrical conductivity are obtained.

These features make TCNAQ compounds industrially useful organic compounds or organic semiconductors. Moreover, by varying the kinds and positions of substituents, varieties can be imparted to the above features. Taking their electrical characteristics as examples, the conductivity values of TCNAQ compounds (A) according to this invention lie in the range of about $10^{-9}$ to $10^{-7}\Omega^{-1}\cdot cm^{-1}$.

Moreover, when, of the above-mentioned substituent groups, a chlorine atom, a hydroxy group or an amino group exists on $C_1$ of TCNAQ, there occurs a resonance (mesomeric effect) which extends the conjugated system of π electrons on the tetracyanoanthraquinodimethane skeleton so that some useful results such as a remarkable increase of electrical conductivity are obtained. The above-mentioned resonance effect is schematically illustrated below.

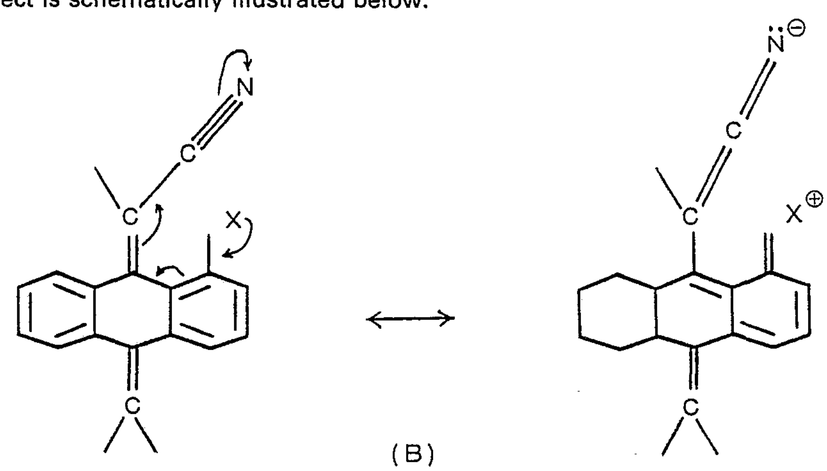

(B)

1) Application and uses

The TCNAQ compounds described above can be used in a variety of applications. Moreover, these compounds are useful as intermediates for the production of other valuable compounds or substances. Furthermore, these compounds or substances may be incorporated or dispersed in other inorganic compounds, organic compounds or high polymer compounds to provide compositions having desirable properties and characteristics. These compounds, substances and compositions generally can be used in a large variety of applications as organo-electronic materials. Desirable results can be obtained by using the compounds of this invention selectively in accordance with the intended use or application.

Some of such applications and uses will be described briefly.

1.1) *Polymers*

Starting from TCNAQ compounds, various polymers having useful properties can be produced. The term "polymer" as used herein means a compound formed as a plurality of tetracyanoanthraquinodimethane skeletons are linked up either directly or through the intermediary of some other group or groups. The term "group" is used herein to mean an atom or a group of atoms.

By way of illustration, a polyether compound can be synthesized by condensing a TCNAQ derivative (A) having at least two hydroxyalkyl groups as substituents. Polyester compounds can also be synthesized by reacting a TCNAQ derivatives (A) having at least 2 hydroxyalkyl groups as substituents with a TCNAQ derivative (A) having at least 2 carboxy or carboxyalkyl groups as substituents. Moreover, a halogenated derivative (A) can be dehalogenated by Ullmann reaction to obtain a polymer formed as a plurality of tetracyanoanthraquinodimethane skeletons are linked together.

1.2) *Charge transfer complexes*

A TCNAQ derivative (A) or a polymer derived therefrom may be doped with a compound capable of acting as an electron donor or acceptor for it to obtain a charge transfer complex. Such electron transfer

complexes have desirable characteristics and, therefore, are of great industrial value.

The above-mentioned TCNAQ compounds or polymers derived therefrom or charge transfer complexes based thereon may be dispersed or otherwise incorporated in other high molecular compounds or the like to provide compositions having very desirable properties.

As preferred examples of said electron donor, there may be mentioned such metal elements as sodium, copper, etc., aromatic compounds, e.g. anthracene, etc., amines and phthalocyanines. As for said electron acceptor, Lewis acids such as arsenic pentafluoride, etc. are especially desirable.

As matrices in which the compounds and derivatives according to this invention are incorporated or dispersed, various resins and other high molecular compounds are especially useful. In such applications, it is preferable to employ a compound (A) having substituents compatible with the high molecular matrix compound, for such a combination ensures a uniform composition or dispersion. For example, when a polyolefin compound is selected as said high molecular compound, an alkylated derivative (A) can be used with great advantage. When an aromatic high molecular compound is selected as the matrix material, it is advantageous to use a phenylated or alkylphenylated derivative (A).

These TCNAQ compounds and polymers and the charge transfer complexes derived therefrom, as well as compositions containing such compounds, polymers or complexes can be used in a large variety of applications as organo-electronic materials. For example, they can be used as dielectric materials, conductors, resistors, thermisters and other semiconductors, photovoltaic materials, and so on.

The method of producing TCNAQ compounds in accordance with this invention will hereinafter be described briefly, reference being made to TCNQ and TCNNQ compounds for comparison's sake. Thus, the methods of producing TCNQ compounds have been described in the literature such as D. S. Acker et al, J. Am. Chem. Soc. *84*, 3370 (1962), R. C. Wheland et al, J. Org. Chem. *40* (21), 3101 (1975) and U.S. Patent 3,115,506, for instance. By way of example TCNQ is produced from a diethyl succinate by converting the starting compound to 1,4-cyclohexanedione and reacting the latter with malonitrile. The process for synthesis of 1,4-cyclohexanedione from diethyl succinate has been described in J. R. Vincent et al, J. Org. Chem., *3*, 603 (1939) and the subsequent process has been described in the above-mentioned paper of D. S. Acker et al.

In the former process by which 1,4-cyclohexanedione is synthesized from diethyl succinosuccinate, the hydrolysis and decarboxylation reaction of diethyl succinosuccinate require the very rigorous conditions of 195 to 200°C. For this reason, the production of TCNQ by the above series of reactions has had to face a considerable difficulty.

The literature of Wheland et al teaches a method of producing TCNQ compounds. In accordance with the method, a benzene derivative, p-xylene derivative or terephthalic acid derivative is used as a starting material to give a p-xylene halide which is further subjected to a complicated series of steps to give a TCNQ compound. Moreover, the tetracyano compound produced as an intermediate in the above series of reactions is only sparingly soluble in the common solvents because of the high polarity of its 4-cyano groups and, therefore, a large amount of solvent is required in the final stage of the process leading to a TCNQ compound.

Thus, the conventional methods for producing TCNQ compounds require either reactions under very severe conditions, a complicated series of reaction steps, or/and use of large amounts of solvents. These requirements are unfavorable not only in terms of reaction time and yield but also from the standpoints of conservation of energy or power for conducting the reactions and the consumption of solvents, for instance.

The method of producing TCNAQ compounds according to this invention are free from the above-mentioned disadvantages of these methods known for the production of TCNQ compounds and are of great industrial value.

The production method according to this invention will be illustrated by way of chemical reaction formula [Formula (C)]. In Formula C, the substituents W, X, Y and Z are selected from among the substituents $Z_1$ through $Z_8$ in Formula (A).

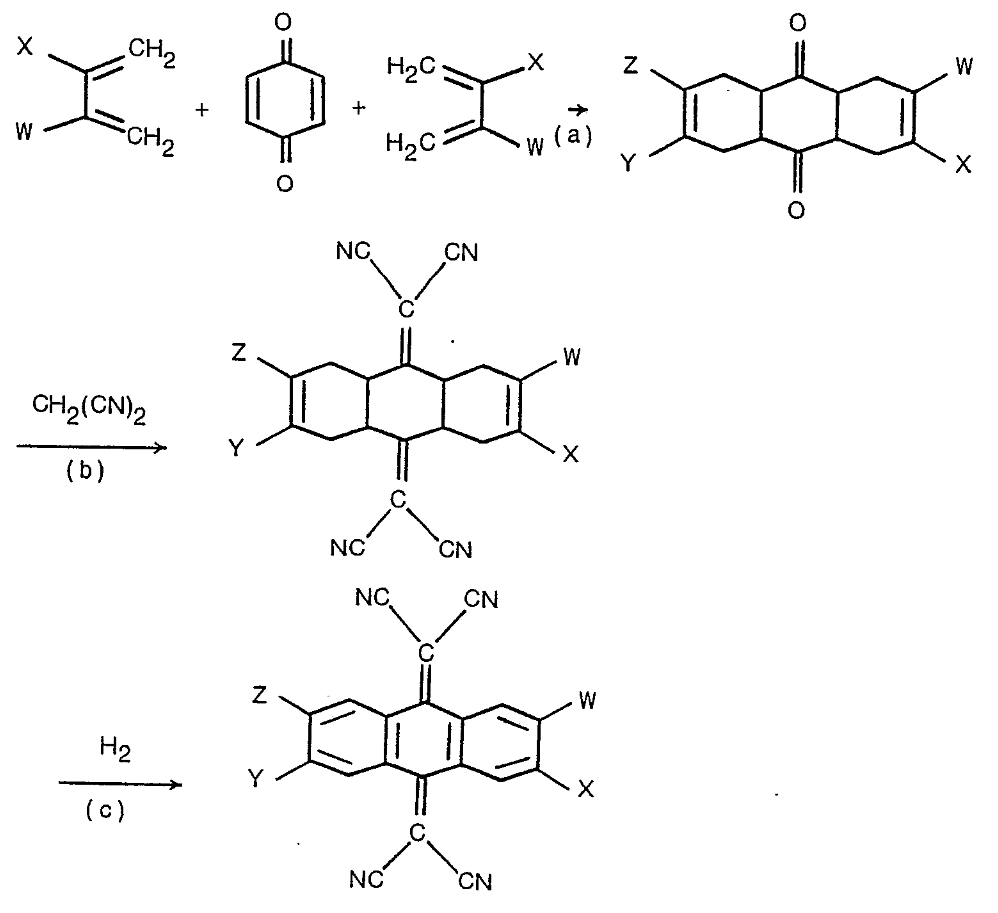


(C)

In the above series of reactions, the Diels-Alder reaction (a) must be conducted under heating at 70 to 80°C but the other reactions proceed satisfactorily under very mild heating or at room temperature, or even at reduced temperature. This means that the method requires only a very low level of energy or power. Moreover, except for the reaction (b) for the introduction of dicyanomethylene groups, all other reactions are simple addition or elimination reactions, and substantially no side reactions occur in any stage of the process. Furthermore, because the method consists of as few as three reaction steps, the desired TCNAQ compound can be obtained in high yield.

In addition, the 1,4,4a,5,8,8a,9a,10a-octahydro-11,11,12,12-tetracyano-9,10-anthraquinodimethane derivative produced in the step (b) is readily soluble in solvents because the high polarity of cyano groups has been mitigated by the two cyclic substituents condensed to the carbon atoms in 4a,9a- and 8a,10a-positions. It is for this reason that a large amount of TCNAQ compound can be produced with use of a small amount of solvent in step (c).

Furthermore, p-benzoquinone and butadiene or its derivative, which are starting materials, are available in quantities and at low cost and this factor also contributes to the economic advantage of this invention.

The following production examples are further illustrative of this invention.

The TCNAQ compounds (A) were produced in the following manner.

i) Synthesis of 1,4,4a,5,8,8a,9a,10a-octahydro-9,10-anthraquinone derivatives

1,4,4a,5,8,8a,9a,10a-octahydro-9,10-anthraquinone compounds were produced from 0.5 mole of p-benzoquinone and 1.2 moles of butadiene or its derivatives.

The butadiene derivatives employed were 2-methylbutadiene, 2,3-dimethylbutadiene, 2-ethylbutadiene, 2,3-diethylbutadiene, 2-propylbutadiene, 2-butylbutadiene, 2-pentylbutadiene, 2-hexylbutadiene, 2-octylbutadiene, 2-phenylbutadiene and 2-(4-methylphenyl)butadiene.

p-Benzoquinone and one of the above-mentioned butadiene derivatives were dissolved in benzene and the solution was refluxed at 70°C. Though varying somewhat with different species of butadiene derivative, the reaction yields were invariably in excess of 85 percent.

ii) Synthesis of TCNAQ compounds

0.2 Mole of each 1,4,4a,5,8,8a,9a,10a-octahydro-9,10-anthraquinone derivative obtained in i) and 0.24 mole of malonitrile were independently dissolved in 300 ml of benzene, followed by addition of 12 ml of acetic acid and 4 ml of ammonium acetate. The mixture was refluxed for 3 hours under constant stirring. The solution was then cooled and filtered and the crystals were recrystallized from acetonitrile to give the corresponding 1,4,4a,5,8,8a,9a,10a-octahydro-11,11,12,12-tetracyano-9,10-anthraquinodimethane derivative.

Of each of these compounds, a 0.1 mole portion was taken and, together with 0.12 mole of bromine, was added to 200 ml of acetonitrile cooled to 0°C. Then, in $N_2$ gas streams, 25 ml of pyridine was added and the solution was stirred at 0°C for an hour. To the reaction mixture was added cold water and the resulting crystals were recovered by filtration and recrystallized from acetonitrile. The TCNAQ compounds produced in the above manner are shown in Table 1. Though varying somewhat with different species of TCNAQ compound, the yields were invariably over 90%. The overall yields of TCNAQ compounds through the above processes i) and ii) were invariably over 75%.

TABLE 1

TCNAQ Compounds

| Butadiene or its derivatives | | TCNAQ Compounds |
| --- | --- | --- |
| W– | X– | |
| H– | $CH_3$– | 2,6–Dimethyl–TCNAQ, 2,7–Dimethyl–TCNAQ |
| $CH_3$ | $CH_3$– | 2,3,6,7–Tetramethyl–TCNAQ |
| H– | $C_2H_5$– | 2,6–Diethyl–TCNAQ, 2,7–Diethyl–TCNAQ |
| $C_2H_5$– | $C_2H_5$– | 2,3,6,7–Tetraethyl–TCNAQ |
| H | $CH_3(CH_2)_2$– | 2,6–Dipropyl–TCNAQ 2,7–Dipropyl–TCNAQ |
| H | $CH_3(CH_2)_3$– | 2,6–Dibutyl–TCNAQ, 2,7–Dibutyl–TCNAQ |
| H | $CH_3(CH_2)_4$– | 2,6–Dipentyl–TCNAQ, 2,7–Dipentyl–TCNAQ |
| H | $CH_3(CH_2)_5$– | 2,6–Dihexyl–TCNAQ, 2,7–Dihexyl–TCNAQ |
| H | $CH_3(CH_2)_7$– | 2,6–Dioctyl–TCNAQ, 2,7–Dioctyl–TCNAQ |
| H | 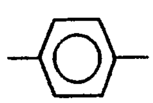 | 2,6–Diphenyl–TCNAQ 2,7–Diphenyl–TCNAQ |
| H | 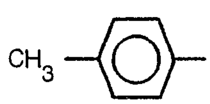 | 2,6-bis(p-Methylphenyl)–TCNAQ 2,7-bis(p-Methylphenyl)–TCNAQ |
| H | H | TCNAQ |

Note) W– and X– represent substituents in Formula (C).

In the above examples, when butadiene derivatives other than 2,3-dimethylbutadiene and 2,3-diethylbutadiene were used as starting materials, two stereoisomers were obtained for each derivative as shown in Table 1. In such cases, the reaction product was separated into the 2,6-di-substituted compound and 2,7-di-substituted compound by routine paper chromatography. Identification of the respective isomers was made by measurement of dipole moment, and the compound showing a dipole moment of zero was identified to be the 2,6-di-substituted compound.

The method of Wheland et al may be advantageously employed to obtain derivatives (A) having substituents in designated positions. This is because the inconstancy of addition reaction in the Diels-Alder system is then eliminated. Moreover, by using a mono-substituted anthracene, there can be obtained a mono-substituted TCNAQ. For example, TCNAQ mono-substituted by an alkyl, amino or other group can be obtained in this manner.

By the above procedure, it is easy to introduce a substituent onto one or more of the C1, C4, C5 and C8 positions. For example, 1,4-dihydroxy-11,11,12,12-tetracyano-9,10-anthraquinodimethane (DOCAQ) and 1-amino-11,11,12,12-tetracyano-9,10-anthraquinodimethane (ACAQ) have such substituents.

In the accompanying drawings,

Figure 1 is a longitudinal cross-sectional view showing a device for measuring electric resistances.

Figure 2 is a diagrammatic representation of the conductivity-temperature characteristics (compressed powder samples) of 11,11,12,12-tetracyano-9,10-anthraquinodimethane (TCNAQ), DOCAQ and ACAQ, when measured using a device as illustrated in Figure 1.

## Claims

1. A 11,11,12,12-tetracyano-9,10-anthraquinodimethane compound of the general formula:

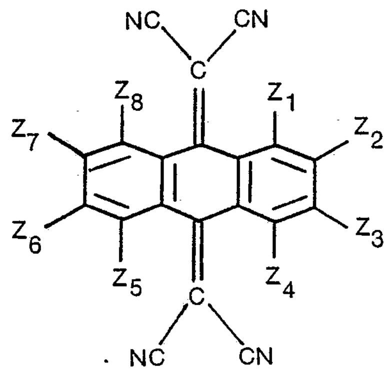

wherein each of $Z_1$, $Z_4$, $Z_5$ and $Z_8$ is H, Cl, hydroxy or amino and each of $Z_2$, $Z_3$, $Z_6$ and $Z_7$ is H, halogen, alkyl, phenyl, alkylphenyl, hydroxyalkyl, carboxyalkyl, hydroxy, amino or carboxy, said alkyl, alkylphenyl, hydroxyalkyl and carboxyalkyl groups each containing up to 8 carbon atoms.

2. A process of preparing a compound according to claim 1 in which $Z_1$, $Z_4$, $Z_5$ and $Z_8$ are all hydrogen, $Z_2$ is the same as one of $Z_6$ and $Z_7$, and $Z_3$ is the same as the other of $Z_6$ and $Z_7$, which comprises carrying out a Diels-Alder reaction between p-benzoquinone and a butadiene of the formula

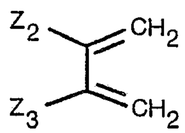

replacing the ketone groups of the resulting Diels-Alder adduct by dicyanomethylene groups and dehydrogenating the resulting tetracyano compound.

3. A polymer comprising a chain having a plurality of units derived from a compound according to claim 1.

4. A charge transfer complex, which comprises a compound according to claim 1 or a polymer according to claim 3, and an electron donor or acceptor.

**Patentansprüche**

1. 11,11,12,12-Tetracyano-9,10-anthrachinodimethan-Verbindung der allgemeinen Formel:

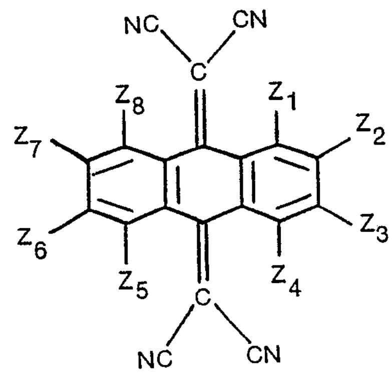

worin bedeuten:
$Z_1$, $Z_4$, $Z_5$ und $Z_8$ jeweils H, Cl, Hydroxy oder Amino und
$Z_2$, $Z_3$, $Z_6$ und $Z_7$ jeweils H, Halogen, Alkyl, Phenyl, Alkylphenyl, Hydroxyalkyl, Carboxyalkyl, Hydroxy, Amino oder Carboxy, wobei die Alkyl-, Alkylphenyl-, Hydroxyalkyl- und Carboxyalkylgruppen jeweils bis zu 8 Kohlenstoffatome enthalten.

2. Verfahren zur Herstellung einer Verbindung nach Anspruch 1, worin $Z_1$, $Z_4$, $Z_5$ und $Z_8$ sämtlich Wasserstoff bedeuten, $Z_2$ die gleiche Bedeutung hat wie eines von $Z_6$ und $Z_7$ und $Z_3$ die gleiche Bedeutung hat wie das andere von $Z_6$ und $Z_7$, umfassend das Durchführen einer Diels-Alder-Reaktion zwischen p-Benzochinon und einem Butadien der Formel

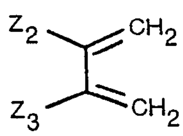

Ersetzen der Keton-Gruppen des resultierenden Diels-Alder-Addukts durch Dicyanomethylen-Gruppen und das Dehydrieren der resultierenden Tetracyano-Verbindung.

3. Polymer, umfassend eine Kette mit einer Vielzahl von Einheiten, die sich von einer Verbindung nach Anspruch 1 ableiten.

4. Charge-Transfer-Komplex, umfassend eine Verbindung nach Anspruch 1 oder ein Polymer nach Anspruch 3 und einen Elektronendonor oder -akzeptor.

**Revendications**

1. Composé de 11,11,12,12-tétracyano-9,10-anthraquinodiméthane caractérisé en ce qu'il répond à la formule générale:

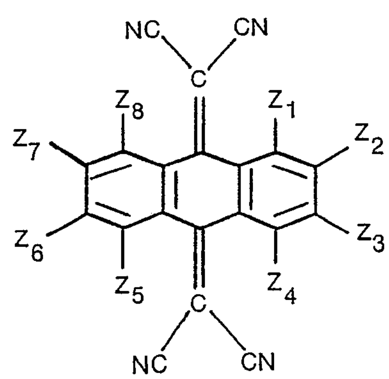

dans laquelle chacun des restes $Z_1$, $Z_4$, $Z_5$ et $Z_8$ est H, Cl, hydroxy ou amino et chacun des restes $Z_2$, $Z_3$, $Z_6$ et $Z_7$ est H, halogène, alkyle, phényle, alkylphényle, hydroxyalkyle, carboxyalkyle, hydroxy, amino ou carboxy, lesdits groupes alkyle, alkylphényle, hydroxyalkyle et carboxyalkyle contenant chacun jusqu'à 8 atomes de carbone.

2. Un procédé de fabrication d'un composé selon la revendication 1, dans laquelle $Z_1$, $Z_4$, $Z_5$ et $Z_8$ sont tous des atomes d'hydrogène, $Z_2$ est le même que l'un des substituants $Z_6$ et $Z_7$, et $Z_3$ est le même que l'autre desdits substituants $Z_6$ et $Z_7$, caractérisé en ce qu'il consiste à effectuer une réaction de Diels-Alder entre la p-benzoquinone et un butadiène de formule

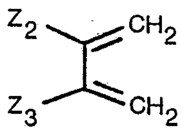

à remplacer les groupes cétone de l'adduct de Diels-Alder résultant par des groupes dicyanométhylène et à déshydrogéner le composé tétracyano résultant.

3. Polymère, caractérisé en ce qu'il comprend une chaîne comportant plusieurs motifs dérivés d'un composé selon la revendication 1.

4. Complexe de transfert de charge, caractérisé en ce qu'il comprend un composé selon la revendication 1 ou un polymère selon la revendication 3 et un donneur ou un accepteur d'électrons.

Fig.1

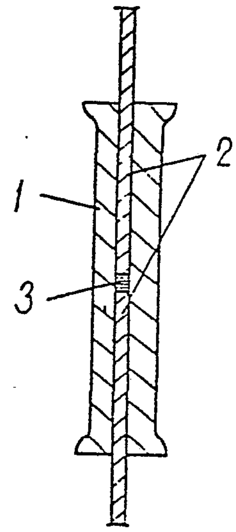
1
2
3

Fig.2

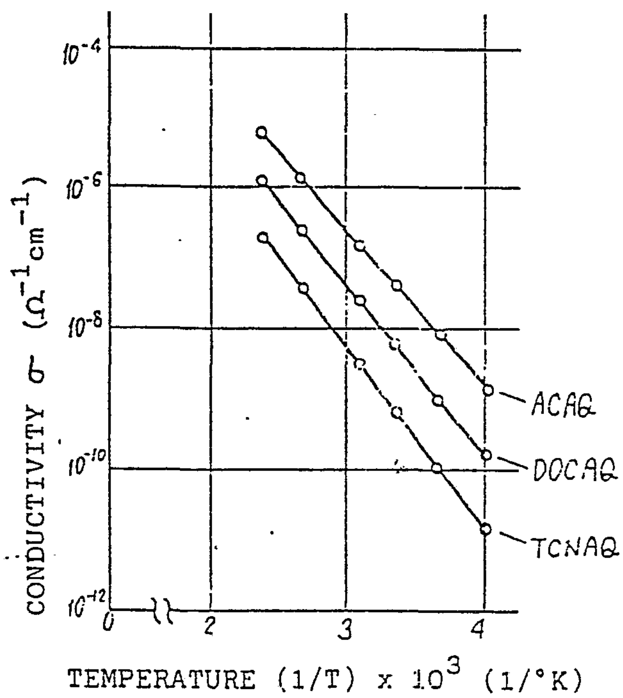
CONDUCTIVITY σ (Ω⁻¹cm⁻¹)
10⁻⁴
10⁻⁶
10⁻⁸
10⁻¹⁰
10⁻¹²
0
2
3
4
ACAQ
DOCAQ
TCNAQ
TEMPERATURE (1/T) x 10³ (1/°K)